# EUROPEAN PATENT APPLICATION

(11) **EP 1 698 365 A2**
(43) Date of publication of application: **06.09.2006**
(21) Application number: 06012233.0
(22) Date of filing: 08.10.2004
(51) Int. Cl.: A61M 5/315

(54) **Injection device for administering a fluid product**

(30) Priority: 16.10.2003 DE 10348186; 16.10.2003 US 687517
(62) Divisional of application: 04761958.0
(71) Applicant: TecPharma Licensing AG, 3401 Burgdorf (CH)
(72) Inventor: Hommann, Edgar, 3257 Grossaffoltern (CH); Himbert, Hans, 16856 Bromma (SE); Vejbrink, Ulrika, 129 40 Hagersten (SE)

(57) **Abstract**

An injection device for administering a fluid product including operating means pivotable in a radial direction relative to the device about a fulcrum arranged laterally on the device, wherein the operating means includes a protrusion which co-operates with dispensing means for dispensing the product via a surface oblique with respect to a longitudinal axis of the device, such that the dispensing means may be moved in an axial direction relative to the device by pivoting the operating means. In some embodiments, the device may further include a moveable releasing element for releasing a dose, wherein the releasing element projects through an opening, the dimensions of the opening limiting movement of the releasing element in accordance with a predetermined dose amount. In some embodiments, the device may further include a shiftable sleeve arranged such that, in one position, the sleeve surrounds an injection needle associated with the device.

## Description

### Background

The present invention relates to injection devices and methods for administering fluid products. More particularly, it relates to an injection device comprising manually operable means for dispensing a fluid product, a residual amount indicator, and a needle protecting means.

Conventional injection devices, including injection pens, are used for administering various medical, pharmaceutical and cosmetic fluid products, for instance insulin, growth hormones, etc. Typically, they comprise a casing in which various means enabling the fluid product to be administered according to various requirements and a container for the fluid product are accommodated, and to which an injection needle is connected. Generally, a means for dispensing the fluid product from the container possesses an operating means to be operated manually, using which a mechanism can be triggered which displaces the product from the container. A dosing means is usually provided, which can set a particular product amount to be dispensed, such that a particular dose is administered when the operating means is triggered. In many cases, an indicator is provided to indicate the size of a dose, a total product amount dispensed or a residual amount of the product remaining in the container.

An exemplary injection device, an injection pen, for self-injection is known from DE 4208677 A1. The disclosed device consists of a sleeve-shaped casing including a carpoule, a cannula and a piston. It further comprises a pushing rod with a push-button, and a dosage setting means. The injection device is accommodated in a storage case. The push-button protrudes out of the injection device at an end opposite the cannula. Administering a product can be triggered by pushing the button axially relative to the body of the device, with the thumb, while the device is held in the hand. The push-button is provided with a tappet which protrudes through a longitudinal slit in the storage case and is visible from the exterior side of the case. With each injection, the protruding end of the pushing rod becomes continuously shorter, such that a residual amount of the product still remaining in the carpoule can be read, by means of a scale, from the position of the tappet arranged on the push-button. Once a dosage has been set, the pen is taken out of the case, for administering the dose. Until the injection device is removed, the storage case forms a protection around the cannula, such that pricking injuries can be prevented.

In WO 96/32973, an elongated medical injection apparatus is described in which a moving wing is provided on one side, said wing serving to trigger administering. To this end, the user clasps the circumference of the injection apparatus and pushes the wing into the interior of the apparatus, such that it performs a circular movement about a fulcrum on the apparatus. By pivoting the wing, an administering mechanism is activated, and simultaneously a dosage indicator is automatically reset to an initial zero position after the injection. A needle cover is provided for an injection needle at one end of the apparatus, said needle cover being slid onto the needle such that it surrounds it. A further cover for the needle is formed by a casing cap which is plugged as a separate part onto the casing of the injection apparatus, over the needle with the needle cover. The casing cap then forms a part of the casing.

One problem not addressed adequately by conventional injection devices is that it may be difficult for users who are restricted in their motor functions to operate an axial push-button type device using only their thumb. Attaching an operating means laterally, however, is often associated with extensive changes to the dispensing or dosing means. Furthermore, it is often not necessary to provide a complex monitoring of the product amount in the container, for example if an identical, fixedly pre-set dosage is always to be administered, as in, for instance, osteoporosis therapy.

It is an object of the invention to provide an injection device comprising means for dispensing the fluid product which can be operated simply and enables a fluid product to be reliably delivered.

It is a further object of the invention to provide an injection device comprising dose release means which can also comprise a residual amount indicator having a simple construction design which reliably indicates a number of dispensing dosages still remaining.
Yet another object of the invention is to provide an injection device comprising a needle protecting means which securely protects against pricking injuries while the device is prepared and used.

### Summary

In one embodiment, the present invention comprises an injection device comprising operating means pivotable in a radial direction relative to the device, wherein the operating means is co-operative with dispensing means via a surface oblique with respect to a longitudinal axis of the device and when the operating means is pivoted, the dispensing means moves in an axial direction relative to the device. In some embodiments, the operating means is pivotable about a fulcrum arranged laterally on the device and, in some embodiments, includes a protrusion co-operative with the dispensing means via the oblique surface.

In one embodiment, the present invention comprises an injection device comprising operating means pivotable in a radial direction relative to the device about a fulcrum arranged laterally on the device, wherein the operating means includes a protrusion co-operative with dispensing means for dispensing a product via a surface oblique with respect to a longitudinal axis of the device, such that the dispensing means is moved in an axial direction relative to the device by pivoting the operating means. In some embodiments, the device may further comprise a moveable dose releasing element for releasing a dose, wherein the releasing element projects through an opening, the dimensions of the opening limiting movement of the releasing element in accordance with a predetermined dose amount. In some embodiments, the device may further comprise a shiftable sleeve arranged such that, in one position, the sleeve surrounds an injection needle associated with the device.

In one embodiment, an injection device for administering a fluid product, as set forth in the invention, comprises casing which may be generally cylindrical. A dispensing means for dispensing the fluid product from a product container, an operating means for operating the dispensing means, a dosing means for releasing a predetermined size of a dosage and an indicator for indicating a product amount can be provided in the casing. The product container, e.g., an ampoule or the like, can be fitted such that it may be inserted into and removed from the casing, such that a new one can be inserted once the container has been emptied. It is also possible to provide a holder for the product container into which the product container is inserted and then accommodated in the injection device. An injection needle is connected to the product container and is attached such that it can be exchanged, in order to be able to use a sterile needle for each injection. The dispensing means may be formed by an advancing element which drives a piston in the product container toward an outlet in the container, such that product can be displaced from the container. The advancing element can be formed by a piston rod which is shifted in the longitudinal direction of the casing of the injection device and thus moves the piston in the product container.

In accordance with one embodiment of the invention, the operating means for the dispensing means is formed by a lever which can be provided laterally on a circumference surface of the casing of the injection device. The fulcrum of the lever is provided on the injection device such that an end of the lever lying away from the fulcrum can be pivoted in a radial direction relative to the injection device. Accordingly, this end can be moved towards and away from the injection device by being pivoted. In one embodiment, the fulcrum is preferably provided laterally on a central area of the injection device. The lever comprises a protrusion, preferably on the end of the lever lying away from the fulcrum, which co-operates with the dispensing means via a surface running obliquely with respect to the longitudinal axis of the injection device, such that the dispensing means, like the piston rod, may be moved in the axial direction by pivoting the lever, i.e., the piston rod can move the piston in the product container. The protrusion pushes the piston rod in the axial direction by sliding along the oblique surface, i.e., by means of the oblique surface, the pivoting movement running in the radial direction generates a force component acting on the piston rod which acts in the axial direction. To this end, the geometric arrangement between the projecting lever, the injection device and the oblique surface is a triangle in which the longest side is formed by the longitudinal axis of the injection device and a somewhat shorter side by the projecting lever, the fulcrum lying between these two sides. The oblique surface as the third side therefore forms an acute angle with the longitudinal axis. When the lever is pivoted, its side of the triangle comes to rest on the side of the longitudinal axis and the piston rod is axially shifted by a distance corresponding to the difference in length of these two sides of the triangle.

In one embodiment, the protrusion of the lever preferably points in the radial direction towards the longitudinal axis of the injection device and preferably acts on an end of the piston rod opposite the piston. The protrusion is then arranged in the casing at an end lying away form the needle. The fulcrum is preferably provided in an area of the casing which as far as possible lies away from the end with the protrusion. This therefore results in a long lever arm with an advantageous transfer of force. In some preferred embodiments, the length of the lever is selected such that all the fingers can come to rest on the lever when clasping the injection device.

The oblique surface can be provided on either or both of the protrusion or the dispensing means. The oblique surface forms a contact surface between the operating means, i.e., the protrusion, and the dispensing means, i.e., the piston rod. The surface of the protrusion which points towards the facing surface of the piston rod may preferably be formed obliquely. It is advantageous if the facing surface of the piston rod is rounded, such that there is a contact point rather than a contact surface with the protrusion. Forming a contact point also reduces the friction when the protrusion slides along the oblique surface. When the lever is pivoted or pushed in the axial direction, the oblique surface is shifted along the contact point. This exerts a force on the piston rod in the axial direction, since pivoting the lever shortens the distance between the contact point and the fulcrum, due to the geometry described above.

In principle, it would also be possible to shift the tip of the protrusion along an oblique surface provided on the dispensing means. Said oblique surface may preferably be arranged on the facing side of the piston rod. The protrusion can then abut the surface via its tip which points in the radial direction to the oblique surface, such that it encloses an angle with said oblique surface. It is also possible for the protrusion to likewise comprise an oblique surface via which it abuts the oblique surface of the piston rod. The bevel of the protrusion with respect to the longitudinal direction of the injection device can exhibit the same or a different inclination than the bevel of the facing side. Due to the contact between the protrusion and the oblique surface of the dispensing means, a force is exerted on the dispensing means in the axial direction when the lever is pivoted, such that a piston can be move in the product container in order to deliver the product.

With the aid of an operating means in accordance with the present invention for the injection device, it is possible to reliably and securely deliver a product from the product container without having to perform a particularly controlled, sensitive hand movement. The injection apparatus can be held by the whole interior surface of a hand and the product is securely dispensed by pressing with all the fingers of the hand. Using an injection device as set forth in the present invention, even users who are restricted in their motor co-ordination can easily administer a fluid product.

In a particularly preferred embodiment of the injection device comprising an operating means as set forth in the present invention, the lever is formed by a long arm which encloses a small angle with the longitudinal axis of the injection device and a protrusion which projects from the arm in the form of a tappet towards the axis of the injection device. The protrusion feeds into the casing of the injection device and comprises a lower side orientated towards the dispensing means and an opposing upper side. The piston rod of the dispensing means points, via its facing surface opposite the piston, towards the lower side of the protrusion.

The facing side of the piston rod and the lower side of the protrusion comprise devices by which the piston rod and the protrusion can be connected to each other such that they can slide. An oblique surface in accordance with the invention is then arranged on or can formed by such a device. In one embodiment, such a connection may be preferably formed by a T-connection in which a T-piece projects from the lower side along the length of the lower side of the protrusion and two hooks aligned opposite each other are provided on the facing surface of the piston rod. The T-bar of the T-piece engages with the hooks on the piston rod and slides within the hooks of the piston rod when the lever is pivoted. In accordance with the invention, the dispensing means of the injection device is operated by the pivoting. Also, due to the sliding connection between the protrusion and the piston rod, the piston rod can likewise be restored in a reverse movement along the longitudinal axis when the lever is restored. The sliding connection can also serve as a guide for the lever and the piston rod when the lever is pivoted.

It is possible in accordance with the present invention to provide the lever of the operating means with a spring which is compressed in the radial direction of the injection device when the lever is pivoted. When the firm grip for administering a product dosage is released, the lever is then restored to its starting position in the opposite direction by the spring force. A leaf spring, whose bending point is arranged near the fulcrum of the lever, may be used. It is also possible to cause the lever and piston rod to be automatically restored using a spring element on the piston rod which is biased with respect to the longitudinal axis of the injection device when the lever is pivoted. Other suitable biasing or urging arrangements may be used.

In accordance with another aspect of an injection device for administering a fluid product as set forth in the present invention, a dosing means for releasing a predetermined size of a dosage comprises a release element for releasing the dosage which projects from the injection device through an opening in the casing of the injection device. The release element can, for example, be formed by a small tappet or a lever extending or orientated radially relative to the main body of the injection device. The opening in the casing can be formed by a square or rectangular cavity or a slit extending along the circumferential direction on one side of the injection device. The lever can be moved in the length of the opening in order to release the dosage. The dimensions of the opening, such as for instance the length of the rectangle or slit in the circumferential direction of the injection device, are selected such that when the release element is moved to release the dosage for a predetermined dosage amount, said releasing movement is limited in accordance with the predetermined dosage. In one embodiment, to release a predetermined dosage the lever is moved from a first stopper on one side of the to a second stopper on the opposite side of the opening. Due to the stoppers, the lever cannot be moved further, and the dimensions of the opening correspod to a predetermined size of a dosage. In one embodiment, the size of the opening is selected such that the pivoting lever can move approximately 90°. In some embodiments, the lever movement may be incremented.

In an injection device comprising a dosing means in accordance with the present invention, a predetermined fixed size of a dosage can be reliably released without a delicate setting movement, such as turning or rotating a dose setting knob, being necessary for this purpose. Even if the release movement is rough, the dosage release is ensured by the stoppers of the lever at the opening in the casing.

Once the predetermined product dosage has been released, the release element of the dosing means can be restored by hand, or automatically recoil, into a starting position at the first stopper, due to a suitable biasing element. Once the product dosage has been administered, the dosing means is then ready to release the next dosage. Using a dosing means as set forth in the present invention, the intention is to enable a fixed, predetermined size of a dosage to be reliably released after each administering of a dosage. It is, however, also possible to provide a device using which the dimensions of the opening in the casing can be changed, such that a different predetermined, fixed size of a dosage can be released. The movement length from the first stopper of the lever at the opening to the second stopper at the opening is changed using such a device.

In one preferred embodiment of the injection device comprising the dosing means as set forth in the present invention, an indicator is provided for indicating a product amount which is still present in the product container and therefore available for further administering. In one embodiment, the indicator comprises a scale with consecutive whole numbers, using which product amount still available can be indicated as the number of discrete dosage units remaining. Whenever the dosing means is operated, i.e., when the lever is moved from the first stopper to the second stopper of the opening, or whenever the dispensing means is operated, i.e., when the operating means is pivoted or retracted, the indicator counts down by one dosage unit. To this end, the scale can be provided on a scale drum which can be rotated in the circumferential direction of the injection device, and arranged behind a window in the casing through which only one number of the scale is visible at a time. The scale drum can then be simultaneously rotated by the movement of the lever when releasing a dosage.

It is, however, also possible to provide an actuator on the lever serving as the operating means of the dispensing means of the injection device, and a grating on the scale drum. Due to the lever pivoting when a dosage is dispensed, the actuator co-operates with the grating and rotates the scale drum in front of the window in the casing forwards by one scale unit. The next lowest number then appears in the window, since one dosage unit less is available in the product container. As the lever of the dispensing means is pivoted or pulled out, the actuator slides over the grating, without the scale being adjusted further. In order to secure the scale drum in the opposite rotational direction, a second latching means can additionally be provided on an adjacent element.

If, in the treatment of a patient, it is necessary to administer a particular dosage amount daily, it is possible to read from the indicator for how many days enough product is present in the product container. To administer the product, it is merely necessary for the user to move the lever of the dosing means from the first stopper to the second stopper of the opening in the casing, place the injection device at the injection point and pivot the lever of the operating means. A blocking means can be provided which prevents the product from being dispensed if the lever has not been moved completely to the second stopper. Advantageously, a green coloration is visible on one side of the lever through the opening in the casing, and a red coloration on the other side. This can make releasing the product dosage optically recognisable, since release is only complete when only the green coloration can be seen in the opening. Once the product dosage has been released, the blocking means releases the dispensing means, and the operating means, i.e., the lever, can be operated by pressing the injection device together in the user's hand, such that the product dosage is administered. In the case of the present invention, the dosage can be set and the product administered using particularly simple hand movements.

In an embodiment of the injection device comprising an operating means as set forth in the present invention and a conventional dosing means, it is in principle also possible to operate an indicator for indicating a product amount using the operating means. A dosing means in accordance with the invention, comprising a release element which protrudes through an opening of predetermined dimensions in the casing, is not necessarily required in order to indicate the product amount. For operating the indicator, the lever of the operating means may comprise an actuator as described above.

### Brief Description of the Drawings

- Figure 1: is a cross-section through a preferred embodiment of an injection device as set forth in the present invention;
- Figure 2,: including Figures 2a-e, depicts embodiments of the present invention, and includes cross-sections (Figures 2a -c) depicting an operating means as set forth in the present invention, in a first and second state, an exploded view (Figure 2d) and a plan view (Figure 2e);
- Figure 3: is a perspective, partially sectional view of an operating means as set forth in the present invention, comprising an indicator for indicating a product amount;
- Figure 4,: including Figures 4a and 4b, is a cross-section through an embodiment of the injection device, comprising an operating means in accordance with the invention as set forth in Figure 3;
- Figure 5: is an exterior view of an embodiment of an injection device, comprising a dosing means in accordance with the present invention;

### Detailed Description

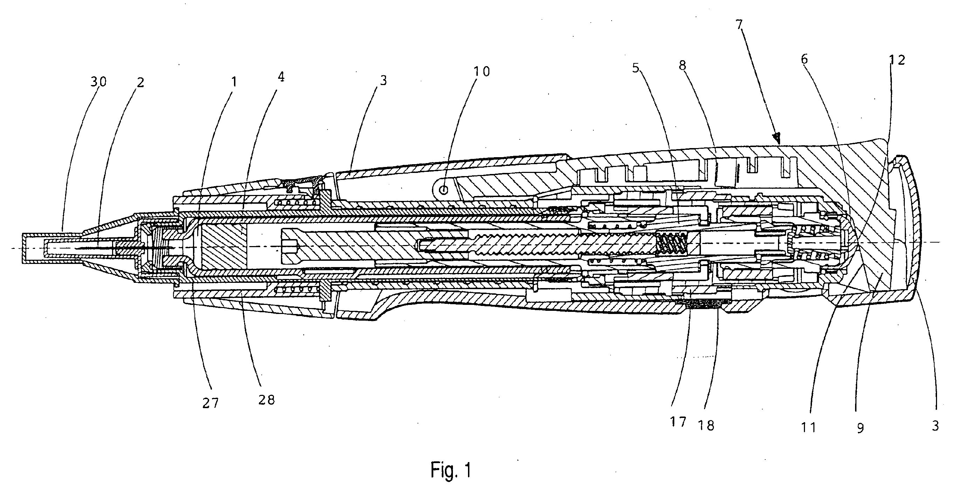

Figure 1 shows an injection device for administering a fluid product, in a first embodiment of the present invention. The injection device comprises a product container 1, to which an injection needle 2 is connected and which is inserted into an elongated casing 3 of the injection device. A piston 4 is arranged in the product container 1, wherein said piston can be moved by a dispensing means towards the outlet comprising the injection needle 2. The dispensing means comprises a number of elements which together serve as a piston rod 5 and by which the piston 4 is moved. The piston rod 5 exhibits a rounded facing surface 6 at an end of the piston rod 5 opposite the piston 4.

The operating means for operating the dispensing means of the injection device shown in Figure 1 is formed as a lever 7 comprising a lever arm 8 and a protrusion 9. A fulcrum 10 of the lever 7 is laterally arranged on the injection device, about in the middle with respect to the length of the injection device. The fulcrum 10 is substantially provided on the circumferential surface of the injection device. The lever 7 can be pivoted in the radial direction of the casing 3 via the fulcrum 10. Accordingly, the lever 7 can be moved towards the longitudinal axis of the injection device. In some embodiments, the lever arm 8 is preferably formed with a length corresponding to the average width of a hand. In some embodiments, the overall length of the lever may be approximately 85 mm, and the exposed length may be 63 mm, but these lengths may be varied as suitable. In some embodiments, the free end of the lever may travel approximately 6 mm, but this may be varied as suitable. In some embodiments, the force applied to the free end of the lever to actuate the device ranges between approximately 15-20 N; this force may be varied as suitable, and may depend on additional factors such as: the diameter of the needle of the device, the friction between the piston and ampule, etc.

The protrusion 9 projects substantially perpendicularly from the lever arm 8 towards the longitudinal axis of the injection device and feeds into the casing 3 at an end of the injection device opposite the needle. On a lower side facing the piston rod 5, the protrusion 9 exhibits an oblique surface 11 which, as described above, runs or extends obliquely with respect to a longitudinal axis of the casing 3 and encloses an acute angle with the longitudinal axis. This means that the oblique surface exhibits an inclination towards the pivoting direction of the lever which is formed sloping towards the lever arm 8. The oblique surface 11 of the protrusion 9 points to the rounded facing surface 6 of the piston rod 5 at an angle, such that it contacts the facing surface 6 at a contact point 12.

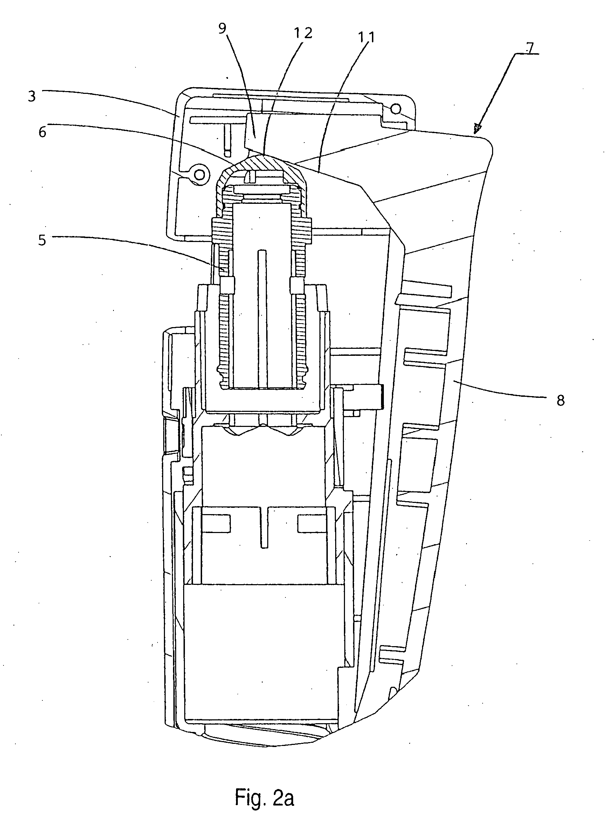

Figure 2a shows the injection device described in Figure 1 in a first state in which the lever 7 is in a position pivoted away from the casing 3 of the injection device. The contact point 12 between the oblique surface 11 of the protrusion 9 and the facing surface 6 of the piston rod 5 is situated in an area near the tip of the protrusion 9. A step 13 is provided on the upper side of the protrusion 9, opposite the oblique surface 11, said step pushing against an edge of the casing 3 and preventing the lever 7 from pivoting further and out of the casing 3. In the first state, the injection device is in a starting position for dispensing a dosage of the fluid product from the product container.

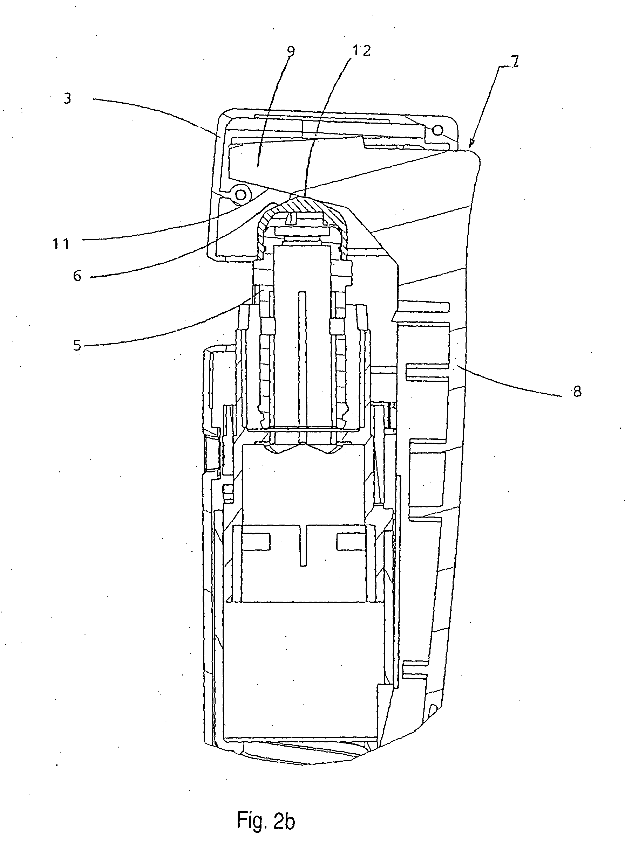

Figure 2b shows the injection device of Figure 1 in a second state in which the lever 7 is pivoted or pushed in the radial direction, into the casing. The contact point 12 between the oblique surface 11 of the protrusion 9 and the facing surface 6 of the piston rod 5 lies further away from the tip of the protrusion 9 than the contact point shown in Figure 2a. Due to the geometry of the triangle formed by the piston rod 5, the lever arm 8 and the oblique surface 11 of the protrusion 9, it is possible when pivoting the lever 7 to generate a force in the direction of the longitudinal axis of the casing, onto the outlet of the product container 1. When the lever 7 is pivoted into the casing 3, the contact point 12 slides along the oblique surface 11, which converts the movement of the protrusion 9 in the radial direction of the casing 3 into a force component in the longitudinal direction of the casing 3. Using the force component in the longitudinal direction of the casing 3, the piston rod 5 can shift the piston towards the outlet of the product container and administer a product dosage.

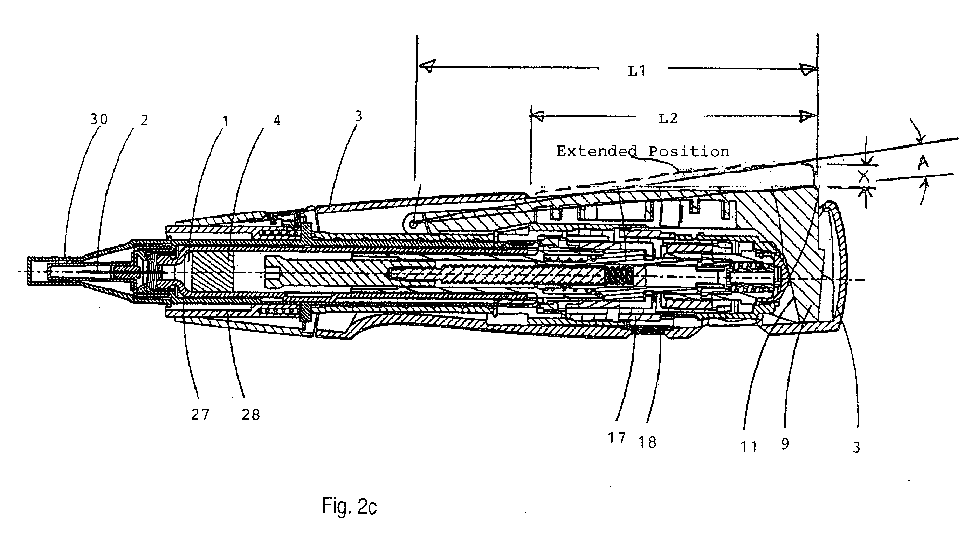

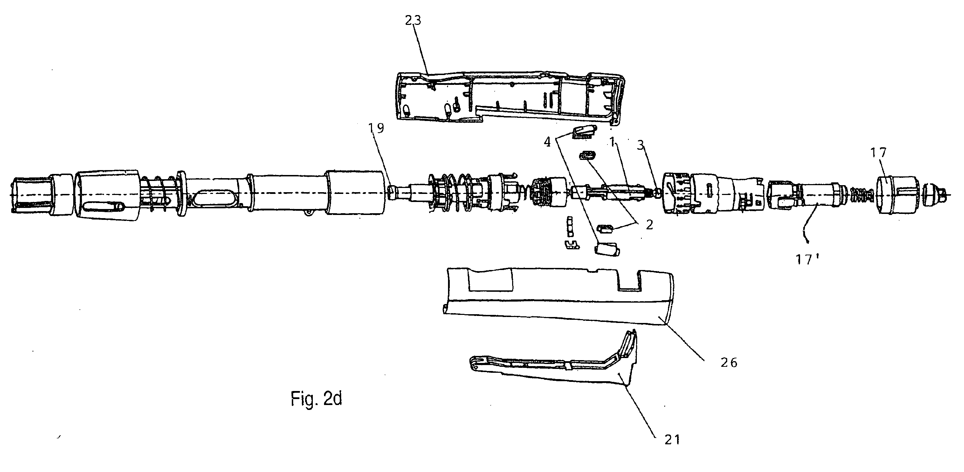

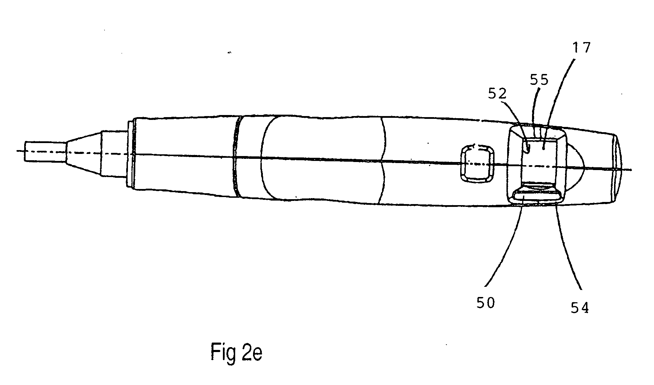

With continued reference to Figure 2, which includes Figures 2a-e , Figures 2c and 2d depict an embodiment wherein an activating member 17 may be provided in order to effect actuation of the dispensing mechanism upon displacement of the side lever 21 from a first position to a second, inwardly retracted position. The activating member also acts as a safety mechanism to prevent disposal of fluid product through the needle upon inadvertent displacement of, or application of force to, the side lever 21. Generally, the activating member 17 works in cooperation with the split nut 2 and split nut sleeve 4, which belong to a holding mechanism for holding or restraining parts of the dispensing mechanism as ampoules are changed, to cause selective engagement and disengagement of the split nut 2 with the threaded drive rod assembly 1, 3, 19 depending on or reflecting the radial position of the activating member 17. More particularly, in one embodiment, the activating member 17 cooperates with a coupling sleeve which is shown in Figure 2d generally next to the activating member 17. Referring to Figure 2e, moving the activating member 17 from the first stop 54 to the second stop 55 results in turning the coupling sleeve, the sleeve 1, the split nut 2 and the split nut sleeve 4 on the threaded rod. The activating member 17 may include a radial projection 50 extending through an opening 52 in the housing 23, 24. The radial projection 50 enables a user of the injection device to position the activating member 17 at a first position, wherein the radial projection 50 abuts a first stop 54, or a second position, wherein the radial projection 50 abuts a second stop 55.

In one embodiment, with the radial projection 50 positioned against the first stop 54, displacement of the side lever 21 has no effect on the dispensing mechanism (e.g., displacement of the threaded rod assembly 1, 3, 19). However, with the radial projection 50 positioned against the second stop 55, the split nut 2 is engaged at a specified longitudinal location with the threaded rod 3. Thus, upon inward displacement of the side lever 21, the threaded rod 3 is advanced or displaced a predetermined distance along the longitudinal axis of the injection device. Additionally, displacement of the side lever 21 may also result in the radial displacement of the activating member 17 such that radial projection 50 is positioned back against the first stop 54 thereby disengaging the split nut 2 from the threaded rod 3 and preventing further actuation of the dispensing mechanism until the radial projection 50 is repositioned by the user to abut the second stop 56. In one embodiment, repositioning of the activation member 17 (i.e., moving the radial projection 50 against the second stop 56) causes the split nut 2 to reengage the threaded rod 3 at a new longitudinal location therealong such that each time the activation member 17 is reset, the threaded rod 3 is longitudinally displaced an additional predetermined distance relative to the threaded rod 3.

In another embodiment, the injection device may be configured such that, upon displacement of the side lever 21 from the first position to the second, inwardly retracted position, the side lever 21 is retained in the second position by a latching member/mechanism until the radial projection 50 of the activating member 17 is displaced from the first stop 54 to the second stop 56 thereby releasing the side lever 21 back to its first position. Such displacement of the radial projection 50 (i.e., from the first stop 54 to the second stop 56) also results in the split nut 2 being released from and longitudinally repositioned relative to, the threaded rod 3. The radial projection 50 must then be displaced back to the first stop 54, thereby reengaging the split nut 2 with the threaded rod 3, in order for further advancement of the threaded rod 3 upon actuation of the side lever 21.

In some embodiments, displacement of the displacement of the lever 21 to an inward position results in an axial movement of the threaded rod assembly and disengages the coupling sleeve of the activating member 17. In some embodiments, turning the activating member 17 while the coupling sleeve is disengaged does not effect the dosing or dispensing mechanism. In some embodiments, when the lever 21 is moved back to its initial outward position, the coupling sleeve is automatically reengaged to the activating member 17. In some embodiments, pushing the side lever 21 again before the activating member 17 is moved once more has no effect on the dispensing mechanism. First the activating member 17 must be moved or displaced (which moves the reengaged coupling sleeve), then the displacement of the side liver 21 results in dispensing the medicament. In some embodiments, in its inward position, the lever 21 is retained to indicate the completion of dispensing the medicament and is released by activating the activating member 17. Generally speaking dosing mechanisms and their functions are known in the art, and such mechanisms and/or their components may be selected as suitable and/or desired for use in the present invention.

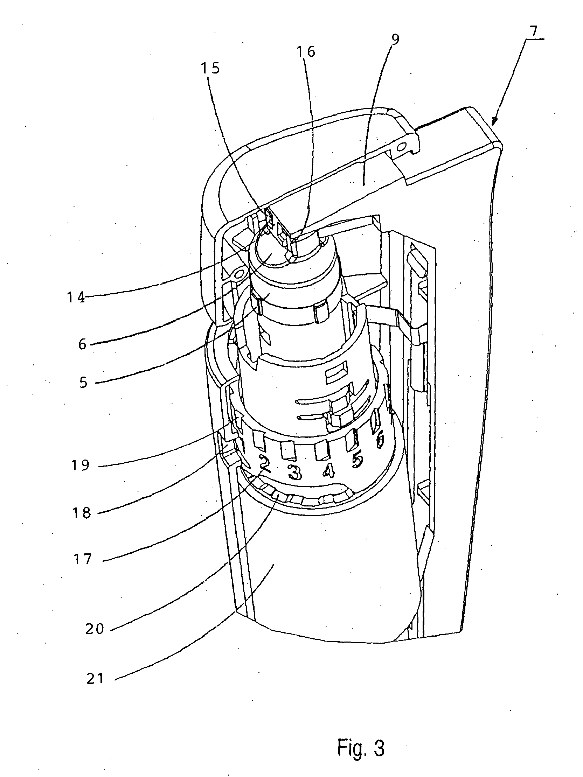

Figure 3 shows a second embodiment of an injection device as set forth in the present invention, in which there is a sliding connection between the protrusion 9 of the lever 7 and the facing surface 6 of the piston rod 5. The sliding connection is formed by a T-connection formed by a T-shaped attachment 14 extending along the oblique surface 11 of the protrusion 9 and two mutually opposing hooks 15 and 16 projecting from the facing surface 6. The hooks 15 and 16 enclose the T-bar of the T-shaped attachment 14 between themselves and the facing surface 6, such that the protrusion 9 cannot be moved from the facing surface 6 in the direction of the longitudinal axis of the casing 3. In the radial direction with respect to the casing 3, however, the protrusion 9 comprising the T-shaped attachment 14 can be slid within the hooks 15 and 16.

When the lever 7 is pivoted into the casing 3, therefore, the T-shaped attachment 14 slides along the facing surface 6, within the hooks 15 and 16. The oblique surface 11 of the protrusion 9 is then formed by the upper side of the T-bar of the T-shaped attachment 14.

Furthermore, an indicator for indicating a product amount in the product container can be provided in an injection device as set forth in the embodiments shown in Figures 1 to 3. As shown in Figures 1 and 3, the indicator can be formed by a scale drum 17 and a window 18 in the casing 3. The scale drum 17 comprises a scale having a graduation of whole numbers of units. Furthermore, a grating 19 is provided on an exterior circumferential surface of the scale drum 17. An actuator 22 pointing radially to the longitudinal axis of the injection device is provided on the lever 7, said actuator pointing towards the scale drum 17 and co-operating with the grating 19. Another latching means 20 may be provided on a facing surface of the scale drum 17, said latching means co-operating with a complementary latching means 20' of a sleeve-shaped element 21 adjacent to the scale drum 17. Due to the scale drum 17 co-operating with the sleeve-shaped element 21 via the latching means 20 and 20', the scale drum 17 is blocked against rotating in one direction, whereas it remains possible for it to rotate in the opposite direction.

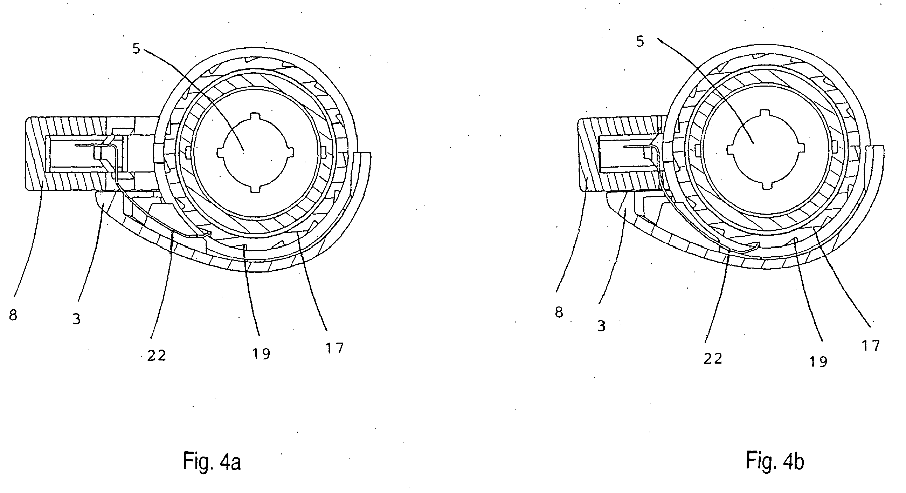

Figure 4a shows the injection device in the first state as set forth in Figure 2a, in a cross-section. Accordingly, the injection device is in a starting position for administering a product dosage, in which the lever 7 is in a position pivoted away. The actuator 22 engages, via its front tip, with the grating 19 of the scale drum 17. Figure 4b shows the injection device in a second position as set forth in Figure 2b, in which the lever 7 is in a position pivoted in. When the lever is pivoted, the actuator 22 is shifted in the radial direction into the casing 3 together with the lever 7, which causes the scale drum to rotate since the actuator 22 pushes against the grating in the rotational direction of the scale drum 17. During a pivoting movement, the scale drum 17 is preferably rotated on by a distance corresponding to the distance between two scale units on the scale drum. It is therefore possible to rotate the scale drum on by one unit with every administering, i.e., with each pivoting of the lever. Preferably, the scale drum counts from a highest numerical value to a lowest numerical value, such that the dosage units remaining in the product container can be read from the indicator. When the lever 7 is pivoted back out of the casing 3 of the injection means, the actuator 22 is pulled across the grating 19, since the tip of the actuator 22 can slide over a bevel of the grating. Also, the scale drum 17 is fixed in an opposite rotational direction by the latching means 20 and 20' of the adjacent sleeve-shaped element 21.

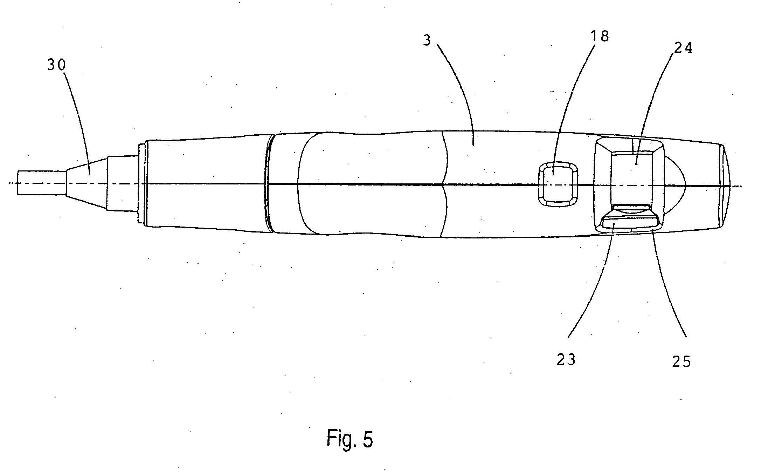

In accordance with another aspect of the present invention, the injection device in a third embodiment shown in Figure 5 comprises a dosing means including a release element in accordance with the present invention. To release a dosage, i.e., to limit the movement of the piston rod in the longitudinal direction in accordance with a desired product dosage, a conventional dosing rotary mechanism can be used. However, such a rotary mechanism is operated in accordance with the invention using a releasing element which projects, in the form of a lever 23, in the radial direction from the injection device. The lever 23 projects outwards through an opening 24 in the casing 3 of the injection device. Limiting the movement of the lever 23 in accordance with a predetermined dosage amount determines the dimensions of the opening 24, i.e., substantially the length of the opening in a circumferential direction of the casing 3. To release a dosage, the lever 23 is moved from a first stopper on a first side 25 of the opening 24 to a second stopper on a second side 26 opposite the first side. Moving the lever 23 from the first stopper to the second stopper releases a pre-determined dosage amount to be administered by the dosing means. In some preferred embodiments, the opening 24 enables the lever 23 to be moved approximately 90° about the longitudinal axis of the injection device.

It is possible to attach the lever 23 to a sleeve which is coloured red on one side of the lever and green on the other side of the lever. This can optically indicate, in a simple way, whether a dosage is ready to be released, since either the red or the green coloration is visible through the opening 24, depending on whether the lever 23 is situated on the first or second side 25 or 26.

A latching device can be provided which does not release a dispensing means for dispensing the fluid product until a dosage has been fully released by means of the dosing means. Using a further latch, for example, the lever 23 can be held at the second stopper, once a dosage has been set, and not released until the dispensing means has been operated and the product dosage administered, such that the lever 23 then recoils to the first stopper.

Embodiments of the present invention, including preferred embodiments, and embodiments of methods of its operation and/or use, including preferred embodiments, have been presented for the purpose of illustration and description. They are not intended to be exhaustive or to limit the invention to the precise forms and steps disclosed. Modifications and variations within the scope of the invention are to be determined by fairly, legally, and equitably interpreting the appended claims.

## Claims

1. An injection device comprising operating means pivotable in a radial direction relative to the device, wherein the operating means is co-operative with dispensing means via a surface oblique with respect to a longitudinal axis of the device and when the operating means is pivoted, the dispensing means moves in an axial direction relative to the device.

2. The injection device according to claim 11, wherein the operating means is pivotable about a fulcrum arranged laterally on the device.

3. The injection device according to claim 12, wherein the operating means comprises a protrusion co-operative with the dispensing means via the oblique surface.

4. An injection device comprising operating means pivotable in a radial direction relative to the device about a fulcrum arranged laterally on the device, wherein the operating means includes a protrusion co-operative with dispensing means via a surface oblique with respect to a longitudinal axis of the device, such that the dispensing means is moved in an axial direction relative to the device by pivoting the operating means.

5. The injection device according to claim 14, further comprising a moveable releasing element for releasing a dose, wherein the releasing element projects through an opening, the dimensions of the opening limiting movement of the setting element in accordance with a predetermined dose amount.
